# FASCICULE DE BREVET EUROPEEN

(11) **EP 2 782 529 B1**
(45) Date de publication et mention de la délivrance du brevet: **06.01.2016**
(21) Numéro de dépôt: 12794445.2
(22) Date de dépôt: 06.11.2012
(51) Int. Cl.: A61F 2/60

(54) **REVETEMENT DE CORPS DE PROTHESE DE MEMBRE, PROTHESE DE MEMBRE ET SON PROCEDE DE FABRICATION**
ABDECKUNG FÜR EINE GLIEDMASSENPROTHESE, GLIEDMASSENPROTHESE UND HERSTELLUNGSVERFAHREN DAFÜR
COVERING FOR A LIMB PROSTHESIS, LIMB PROSTHESIS, AND MANUFACTURING METHOD THEREOF

(30) Priorité: 21.11.2011 FR 1160571
(43) Date de publication de la demande: 01.10.2014
(73) Titulaire: Aqualeg, 44300 Nantes (FR)
(72) Inventeur: RAUCH, Frédéric, F-44000 Nantes (FR)
(74) Mandataire: Benech, Frédéric
(86) Numéro de dépôt international: PCT/FR2012/052560
(87) Numéro de publication internationale: WO 2013/076399

(56) Documents cités:
- EP-A2- 0 985 388
- DE-A1- 4 125 635
- GB-A- 2 067 074
- GB-A- 2 357 725
- US-A- 2 464 391

## Description

La présente invention concerne un revêtement de corps de prothèse de membre en vue d'un habillage du corps de prothèse, une prothèse de membre dont le corps de prothèse est revêtu d'un tel revêtement, et un procédé de fabrication d'un tel revêtement.

Elle concerne plus particulièrement un revêtement tubulaire comprenant une superposition de couches dont au moins une est une couche de silicone.

Les revêtements, dits cosmétiques de corps de prothèse, encore appelés peau ou étui, sont bien connus à ceux versés dans cet art et sont destinés à protéger le corps de prothèse et à lui conférer un aspect esthétique plus agréable à l'oeil comme l'illustre le brevet GB-2.067.074 dans lequel un tel revêtement est réalisé à l'aide de deux couches de silicone superposées, la couche externe étant une couche transparente qui vient à recouvrement d'une couche dite interne de silicone teintée couleur peau dans la masse. Il résulte de cette conception plusieurs inconvénients. Le fait de teinter dans la masse le silicone confère au produit fini un aspect souvent éloigné de l'aspect naturel de la peau humaine. En outre, la flexion de la prothèse a pour effet de fragiliser le silicone jusqu'à déchirement. Si le silicone est trop rigide, il a aussi pour inconvénient de contrarier la flexion et donc la marche du patient.

L'art antérieur le plus proche est divulgué par GB 2 357 725.

Un but de la présente invention est donc de proposer un revêtement du type précité dont la conception permet de se rapprocher de l'apparence de la peau humaine sans nuire à la résistance mécanique dudit revêtement et sans gêner la flexion.

A cet effet, l'invention a pour objet un revêtement de corps de prothèse de membre en vue d'un habillage dudit corps de prothèse, ledit revêtement étant un revêtement tubulaire comprenant une superposition de couches dont au moins une est une couche de silicone, caractérisé en ce que ledit revêtement, dont la ou au moins une couche de silicone est une couche de silicone transparent ou translucide qui forme la couche externe du revêtement, comprend en outre au moins deux couches de tissu élastique teinté, de préférence de teinte différente, au moins la couche de tissu élastique la plus proche de la couche externe étant une couche perméable à la lumière.

La superposition d'une couche de silicone transparente ou translucide et d'une couche de tissu élastique permet de conférer à ladite couche de silicone sa résistance mécanique. La superposition de deux couches de tissu élastique permet de jouer sur la perméabilité à la lumière et la transparence qui résulte d'au moins l'une des couches de tissu pour obtenir, par superposition des couches de tissu teintées généralement de manière différente, un grand nombre de combinaisons et un effet plus réaliste proche de l'apparence de la peau humaine. L'objectif est donc, en superposant plusieurs teintes de couleurs, d'obtenir, par effet de transparence, une couleur ou teinte finale proche de la couleur peau souhaitée. Il doit être noté que le terme "teinté" est un équivalent du terme "coloré". Un tissu élastique teinté est donc un tissu coloré. Deux tissus de teinte différente sont des tissus de couleur différente. Un tissu élastique teinté peut être teinté dans la masse et/ou en surface.

De préférence, le revêtement comprend au moins deux couches de silicone entre lesquelles les deux ou au moins deux couches de tissu élastique teinté sont disposées. Cette combinaison est particulièrement intéressante lorsque le revêtement est destiné à une prothèse devant aller dans l'eau pour obtenir une étanchéité à l'eau et une résistance mécanique suffisantes de ladite prothèse sans nuire à l'esthétique de cette dernière.

Généralement, la couche de silicone, autre que la couche externe de silicone, est une couche opaque formant écran.

De préférence, la superposition de couches comprend, depuis l'extérieur en direction de l'intérieur du revêtement, au moins une couche externe de silicone transparent ou translucide, deux couches de tissu élastique, une couche de silicone et une couche de tissu élastique.

Indépendamment du nombre de couches intermédiaires, la couche externe est une couche de silicone transparente ou translucide tandis que la couche interne du revêtement est une couche de tissu élastique.

De préférence, le revêtement comprend, interposée entre deux couches de tissu élastique teinté, au moins une couche discontinue de peinture, de préférence sous forme de brouillard. La présence de ce brouillard permet de conférer audit revêtement l'aspect irrégulier de la peau.

De préférence, le tissu élastique d'au moins l'une des couches de tissu élastique est un tissu à mailles comprenant au moins un polyamide.

Généralement, au moins l'une, de préférence chaque couche de tissu élastique présente un titrage du fil compris dans la plage 1.10⁻³ à 3,33.10⁻³ g/m, de préférence 1,33.10⁻³ à 2,77.10⁻³ g/m.

De préférence, ledit revêtement étant un revêtement de corps de prothèse fémorale, ledit revêtement comprend dans la zone située au dessus du genou un pli de réserve d'étirement

L'invention a encore pour objet une prothèse de membre comprenant au moins un corps de prothèse revêtu d'un revêtement apte à être enfilé sur ledit corps, caractérisé en ce que ledit revêtement est du type précité.

L'invention a encore pour objet un procédé de fabrication d'un revêtement de corps de prothèse de membre du type précité, en vue d'un habillage dudit corps, caractérisé en ce que ledit procédé comprend au moins :
a) une étape au cours de laquelle on enfile sur un moule positif du membre à reconstituer au moins une couche de tissu élastique sous forme de manchon,
b) une étape au cours de laquelle on revêt la couche de tissu élastique dernièrement enfilée d'une couche de silicone,
et en ce qu'on répète l'étape a) au moins deux fois et l'étape b) au moins une fois en vue de l'obtention d'une superposition de couches comprenant depuis l'extérieur en direction de l'intérieur du revêtement au moins une couche externe de silicone, transparent ou translucide, au moins deux couches de tissu élastique une couche de silicone et au moins une couche de tissu élastique.

L'invention sera bien comprise à la lecture de la description suivante d'exemples de réalisation, en référence aux dessins annexés dans lesquels :
la figure 1 représente une vue schématique en coupe dans l'épaisseur d'un revêtement ;
la figure 2 représente une vue en transparence d'une prothèse fémorale équipée d'un revêtement conforme à l'invention.

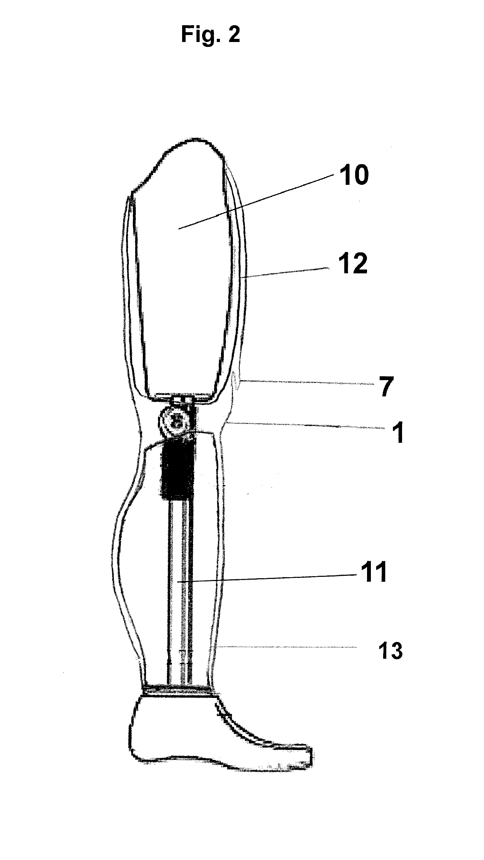

Comme mentionné ci-dessus, le revêtement 1, objet de l'invention, est plus particulièrement destiné à venir à recouvrement d'un corps 10 de prothèse de membre en vue d'un habillage dudit corps 10 de prothèse. Dans l'exemple représenté à la figure 2, la prothèse est une prothèse fémorale dont le corps 10 de prothèse comprend deux coques dites l'une, supérieure et représentée en 12, l'autre, inférieure et représentée en 13. La coque 13 inférieure ainsi que la coque 12 supérieure viennent à recouvrement d'une structure 11 endo-squelettique. Cette structure 11 endo-squelettique comprend ici une tige recouverte par la coque 13 inférieure, cette tige se prolongeant au-delà de la coque inférieure par une articulation reliant lesdites coques entre elles. Le revêtement 1, objet de l'invention, est enfilé sur le corps 10 de prothèse. Ce revêtement se présente à la manière d'un manchon sous forme d'un revêtement tubulaire ouvert à chacune de ses extrémités. Ce revêtement est formé d'une pluralité de couches superposées. La couche 2 externe de ce revêtement est une couche de silicone transparent ou translucide. Une couche de silicone transparent est préférée. Ce revêtement 1 comprend en outre au moins deux couches 41, 42 de tissu élastique teinté, généralement de teinte différente, au moins la couche 41 de tissu élastique la plus proche de la couche 2 externe étant une couche perméable à la lumière. Chaque couche de tissu élastique se présente également sous forme d'un corps tubulaire ou manchon. Généralement, la teinte de base d'au moins l'une des couches de tissu élastique correspond à la teinte de peau du porteur de la prothèse.

Dans l'exemple représenté, le revêtement 1 comprend deux couches 2, 3 de silicone entre lesquelles des couches 41, 42, 43, 44 de tissu élastique teinté sont disposées. La présence de ces deux couches de silicone prenant en sandwich les couches de tissu élastique permet d'éviter une imprégnation des tissus par capillarité par exemple dans le cas d'activité aquatique prolongée.

De préférence, la couche 3 interne de silicone est une couche opaque formant un fond d'écran de l'image formée par la superposition des couches de tissu élastique disposées entre lesdites couches 2, 3 de silicone. Lorsque la deuxième couche 3 de silicone est présente, comme dans la figure 2, cette couche 3 de silicone est doublée intérieurement d'au moins une couche de tissu élastique. En l'occurrence, dans l'exemple représenté, le revêtement 1 présente, côté intérieur de la couche 3 de silicone, deux couches 45, 46 de tissu élastique dont la teinte est indifférente. Par contre, la teinte de la succession de couches de tissu élastique s'étendant derrière la couche externe de silicone transparent confère audit revêtement sa couleur. Chaque couche 41, 42, 43, 44 de tissu élastique apporte sa contribution à la teinte ou couleur définitive dudit revêtement qui est généralement proche de la couleur de la peau du patient. Les teintes de chaque couche de tissu élastique ne peuvent donc pas être choisies au hasard de même que l'ordre, le nombre et le titrage des couches de tissu élastique qui sont fonction de l'aspect esthétique souhaité. Généralement, chaque couche de tissu élastique est un tissu à mailles comprenant au moins un polyamide. A titre d'exemple, on peut utiliser un tissu élastique en polyamide ou en polyamide et élasthanne ou bien encore en polyamide, polyuréthanne et élasthanne. Bien évidemment, d'autres combinaisons peuvent être envisagées. Les tissus élastiques encore appelés bas ou collants vendus par la Société Calzificio Mura S.P.A. sous la référence Myto donnent entière satisfaction.

De préférence, chaque couche 41 à 46 de tissu élastique présente un titrage du fil compris dans la plage 1.10⁻³ à 3,33.10⁻³ g/m, de préférence 1,33.10⁻³ à 2,77.10⁻³ g/m. Exprimé en denier, le titrage est compris entre 9 et 30 deniers, de préférence entre 12 et 25 deniers. La teinte de chaque couche de tissu peut varier à l'intérieur d'une large plage de couleurs. Ainsi, il peut être superposé un tissu 15 deniers composé de 90 % de polyamide et de 10 % d'élasthanne présentant une teinte exprimée en pantone référencée 4665 C et un tissu 20 deniers, 100 % polyamide dont la teinte pantone porte la référence 4655 C. Pour accroître la ressemblance avec l'aspect de la peau humaine, de préférence, le revêtement 1 comprend entre deux couches 41, 42 de tissu élastique teinté, au moins une couche 5 discontinue de peinture, de préférence sous forme de brouillard. A nouveau, le nombre de couches de peinture et leur disposition dans la superposition de couches peuvent influer sur l'aspect final du revêtement. Généralement, la peinture est pulvérisée en brouillard lors de la fabrication du revêtement sur la couche de tissu élastique pour former une couche perméable à la lumière à la surface de ladite couche de tissu élastique. Enfin, il peut être prévu, entre deux couches 41, 42 de tissu élastique ou entre la couche 2 externe de silicone et une couche de tissu élastique au moins une couche 6 de faux poils. Ces faux poils peuvent adhérer à la couche de tissu élastique par électricité statique ou par collage.

Pour parfaire le revêtement 1, dans le cas où ce revêtement est un revêtement de corps de prothèse fémorale, ledit revêtement 1 comprend dans la zone située au dessus du genou un pli 7 de réserve d'étirement

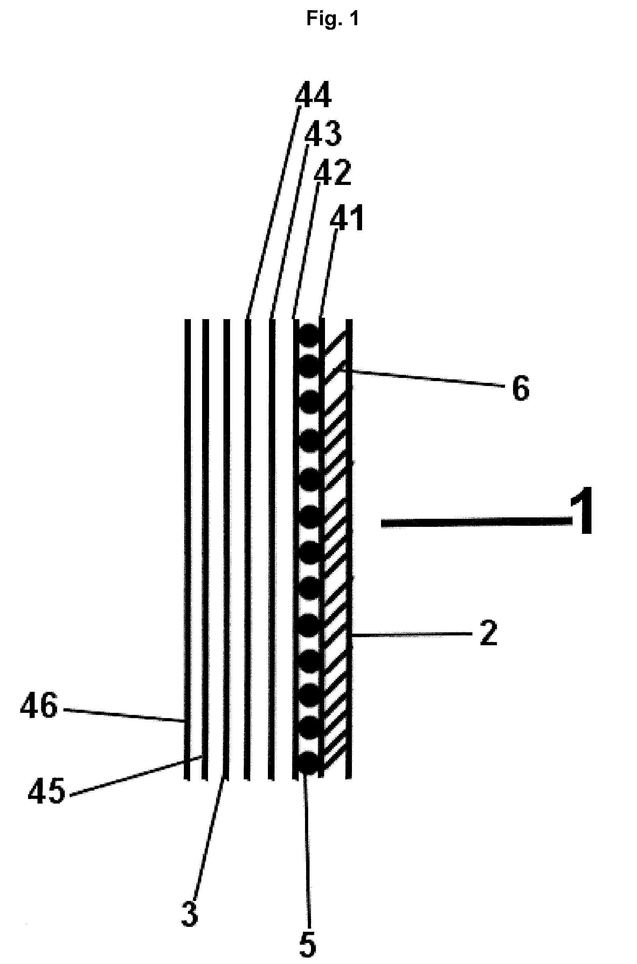

Pour la fabrication d'un revêtement de corps de prothèse conforme à la figure 2, on procède comme suit. Dans une première étape, on enfile, sur un moule positif du membre à reconstituer, au moins une couche 46 de tissu élastique sous forme de manchon. On enfile, sur ce même moule positif, une deuxième couche 45 de tissu élastique, toujours sous forme de manchon, venant se superposer à la couche 46 de tissu élastique. On revêt ensuite cet ensemble d'une couche 3 de silicone. Le silicone peut être appliqué par projection ou enduction manuelle. On reprend ensuite la superposition de couches de tissu élastique enfilées chacune sur le moule positif grâce à leur présentation sous forme de manchon. On enfile ainsi successivement des couches de tissu élastique représentées sous la référence 44, 43, 42 et 41 à la figure 1. A chaque fois, les couches de tissus élastique utilisées peuvent être de titrage, de teinte ou de composition différents. Entre les couches 42 et 41, on procède à une pulvérisation de peinture. On revêt ensuite l'ensemble d'une couche 2 externe de silicone transparent. A nouveau, cette application du silicone peut s'opérer par projection ou enduction manuelle. On rajoute également, entre la dernière couche de tissu élastique 41 et la couche 2 externe de silicone transparent, une couche 6 de faux poils. Une fois le durcissement du silicone opéré à température ambiante, la fabrication du revêtement est achevée.

## Revendications

1. Revêtement (1) de corps (10) de prothèse de membre en vue d'un habillage dudit corps (10) de prothèse, ledit revêtement (1) étant un revêtement tubulaire comprenant une superposition de couches dont au moins une est une couche (2) de silicone transparent ou translucide qui forme la couche externe du revêtement (1), characterisé en ce que ledit revêtement comprend en outre au moins deux couches (41, 42, 43, 44) de tissu élastique teinté, de préférence de teinte différente, au moins la couche (41) de tissu élastique la plus proche de la couche (2) externe étant une couche perméable à la lumière.

2. Revêtement (1) selon la revendication 1,
**caractérisé en ce que** le revêtement (1) comprend au moins deux couches (2, 3) de silicone entre lesquelles les deux, ou au moins deux, couches (41, 42, 43, 44) de tissu élastique teinté sont disposées.

3. Revêtement (1) selon la revendication 2,
**caractérisé en ce que** la couche (3) de silicone autre que la couche (2) externe de silicone est une couche opaque formant écran.

4. Revêtement (1) selon l'une des revendications précédentes,
**caractérisé en ce que** la superposition de couches comprend, depuis l'extérieur en direction de l'intérieur du revêtement (1), au moins une couche (2) externe de silicone transparent ou translucide, au moins deux couches (41, 42) de tissu élastique, une couche (3) de silicone et au moins une couche (45) de tissu élastique.

5. Revêtement (1) selon l'une des revendications précédentes,
**caractérisé en ce qu'**il comprend, interposée entre deux couches (41, 42) de tissu élastique teinté, au moins une couche (5) discontinue de peinture de préférence sous forme de brouillard.

6. Revêtement (1) selon l'une des revendications précédentes **caractérisé en ce qu'**il comprend, entre deux couches (41, 42) de tissu élastique ou entre la couche (2) externe de silicone et une couche (41) de tissu élastique, au moins une couche (6) de faux poils.

7. Revêtement (1) selon l'une des revendications précédentes,
**caractérisé en ce que** le tissu élastique d'au moins l'une des couches (41, 42, 43, 44, 45, 46) de tissu élastique est un tissu à mailles comprenant au moins un polyamide.

8. Revêtement (1) selon l'une des revendications précédentes,
**caractérisé en ce qu'**au moins l'une, de préférence chaque couche (41 à 46) de tissu élastique présente un titrage du fil compris dans la plage 1.10⁻³ à 3,33.10⁻³ g/m, de préférence 1,33.10⁻³ à 2,77.10⁻³ g/m.

9. Revêtement (1) selon l'une des revendications précédentes,
**caractérisé en ce que** ledit revêtement (1) étant un revêtement de corps de prothèse fémorale, ledit revêtement (1) comprend, dans la zone située au dessus du genou, un pli (7) de réserve d'étirement.

10. Prothèse de membre comprenant au moins un corps (10) de prothèse revêtu d'un revêtement (1) apte à être enfilé sur ledit corps (10),
**caractérisé en ce que** ledit revêtement (1) est conforme à l'une des revendications 1 à 9.

11. Procédé de fabrication d'un revêtement (1) de corps (10) de prothèse de membre conforme à l'une des revendications 1 à 9, en vue d'un habillage dudit corps (10), **caractérisé en ce que** ledit procédé comprend au moins :
a) une étape au cours de laquelle on enfile sur un moule positif du membre à reconstituer au moins une couche (45, 46) de tissu élastique sous forme de manchon,
b) une étape au cours de laquelle on revêt la couche (45) de tissu élastique dernièrement enfilée d'une couche (3) de silicone,
et **en ce qu'**on répète l'étape a) au moins deux fois et l'étape b) au moins une fois en vue de l'obtention d'une superposition de couches comprenant depuis l'extérieur en direction de l'intérieur du revêtement (1) au moins une couche (2) externe de silicone, transparent ou translucide, au moins deux couches (41, 42, 43, 44) de tissu élastique, une couche de silicone (3) et au moins une couche (45, 46) de tissu élastique.

## Patentansprüche

1. Abdeckung (1) für den Körper (10) einer Gliedmaßenprothese für eine Verkleidung des Prothesenkörpers (10), wobei die Abdeckung (1) eine rohrförmige Abdeckung mit übereinander angeordneten Schichten ist, von denen mindestens eine Schicht (2) aus durchsichtigem oder durchscheinendem Silikon ist, welche die äußere Schicht der Abdeckung (1) bildet,
**dadurch gekennzeichnet,**
**dass** die Abdeckung ferner mindestens zwei Schichten (41, 42, 43, 44) aus vorzugsweise mit einem unterschiedlichen Farbton gefärbtem elastischem Gewebe umfasst, wobei mindestens die Schicht (41) aus elastischem Gewebe, welche der äußeren Schicht (2) am nächsten liegt, eine lichtdurchlässige Schicht ist.

2. Abdeckung (1) nach Anspruch 1,
**dadurch gekennzeichnet,**
**dass** die Abdeckung (1) mindestens zwei Silikonschichten (2, 3) umfasst, zwischen denen die zwei bzw. mindestens zwei Schichten (41, 42, 43, 44) aus gefärbtem elastischem Gewebe angeordnet sind.

3. Abdeckung (1) nach Anspruch 2,
**dadurch gekennzeichnet,**
**dass** die Silikonschicht (3), bei der es sich nicht um die äußere Schicht (2) handelt, eine undurchsichtige Sperrschicht ist.

4. Abdeckung (1) nach einem der vorhergehenden Ansprüche,
**dadurch gekennzeichnet,**
**dass** die übereinander angeordneten Schichten von außen zum Inneren der Abdeckung (1) mindestens eine äußere Schicht (2) aus durchsichtigem oder durchscheinendem Silikon, mindestens zwei Schichten (41, 42) aus elastischem Gewebe, eine Silikonschicht (3) und mindestens eine Schicht (45) aus elastischem Gewebe umfassen.

5. Abdeckung (1) nach einem der vorhergehenden Ansprüche,
**dadurch gekennzeichnet,**
**dass** sie zwischen zwei Schichten (41, 42) aus gefärbtem elastischen Gewebe angeordnet mindestens eine diskontinuierliche Farbschicht (5) vorzugsweise in Nebelform umfasst.

6. Abdeckung (1) nach einem der vorhergehenden Ansprüche,
**dadurch gekennzeichnet,**
**dass** sie zwischen zwei Schichten (41, 42) aus elastischem Gewebe oder zwischen der äußeren Silikonschicht (2) und einer Schicht (41) aus elastischem Gewebe mindestens eine Kunsthaarschicht (6) umfasst.

7. Abdeckung (1) nach einem der vorhergehenden Ansprüche,
**dadurch gekennzeichnet,**
**dass** das elastische Gewebe mindestens einer der Schichten (41, 42, 43, 44, 45, 46) aus elastischem Gewebe ein Gewirke mit mindestens einem Polyamid ist.

8. Abdeckung (1) nach einem der vorhergehenden Ansprüche,
**dadurch gekennzeichnet,**
**dass** mindestens die eine, vorzugsweise jede Schicht (41 bis 46) aus elastischem Gewebe einen Fadentiter in dem Bereich von 1.10⁻³ bis 3,33.10³ g/m, vorzugsweise von 1,33⁻³.10 bis 2,77.10⁻³ g/m aufweist.

9. Abdeckung (1) nach einem der vorhergehenden Ansprüche,
**dadurch gekennzeichnet,**
**dass** die Abdeckung (1) eine Abdeckung für den Körper einer femoralen Prothese ist, wobei die Abdeckung (1) in dem Bereich über dem Knie eine Streckfalte (7) umfasst.

10. Gliedmaßenprothese mit mindestens einem Prothesenkörper (10) mit einer Abdeckung (1), die geeignet ist, auf den Körper (10) aufgeschoben zu werden,
**dadurch gekennzeichnet,**
**dass** die Abdeckung (1) gemäß einem der Ansprüche 1 bis 9 ausgestaltet ist.

11. Verfahren zur Herstellung einer Abdeckung (1) für den Körper (10) einer Gliedmaßenprothese gemäß einem der Ansprüche 1 bis 9 für eine Verkleidung des Körpers (10),
**dadurch gekennzeichnet,**
**dass** das Verfahren mindestens folgende Schritte umfasst:
a) einen Schritt, bei dem mindestens eine Schicht (45, 46) aus elastischem Gewebe in Form einer Hülse auf einen positive Abdruck der wiederherzustellenden Gliedmaße aufgeschoben wird,
b) einen Schritt, bei dem die zuletzt aufgeschobene Schicht (45) aus elastischem Gewebe mit einer Silikonschicht (3) überzogen wird,
und **dass** Schritt a) mindestens zwei Mal wiederholt wird und Schritt b) mindestens ein Mal wiederholt wird, um übereinander angeordnete Schichten zu erhalten, welche von außen zum Inneren der Abdeckung (1) mindestens eine äußere Schicht (2) aus durchsichtigem oder durchscheinendem Silikon, mindestens zwei Schichten (41, 42, 43, 44) aus elastischem Gewebe, eine Silikonschicht (3) und mindestens eine Schicht (45, 46) aus elastischem Gewebe umfassen.

## Claims

1. Covering (1) for a limb prosthesis body (10), which is intended to dress said prosthesis body (10), said covering (1) being a tubular covering comprising a superposition of layers, of which at least one is a layer (2) of transparent or translucent silicone which forms the external layer of the covering (1), **characterised in that** said covering additionally comprises at least two layers (41, 42, 43, 44) of coloured elastic fabric, preferably of different colours, at least the layer (41) of elastic fabric closest to the external layer (2) being a layer which is permeable to light.

2. Covering (1) according to claim 1, **characterised in that** the covering (1) comprises at least two layers (2, 3) of silicone, between which the two, or at least two, layers (41, 42, 43, 44) of coloured elastic fabric are disposed.

3. Covering (1) according to claim 2, **characterised in that** the layer (3) of silicone other than the external layer (2) of silicone is an opaque screen-forming layer.

4. Covering (1) according to any of the preceding claims, **characterised in that** the superposition of layers comprises, from the outside towards the inside of the covering (1), at least one external layer (2) of transparent or translucent silicone, at least two layers (41, 42) of elastic fabric, a layer (3) of silicone and at least one layer (45) of elastic fabric.

5. Covering (1) according to any of the preceding claims, **characterised in that** it comprises, interposed between two layers (41, 42) of coloured elastic fabric, at least one non-continuous layer (5) of paint, preferably in the form of a mist.

6. Covering (1) according to any of the preceding claims, **characterised in that** it comprises, between two layers (41, 42) of elastic fabric or between the external layer (2) of silicone and a layer (41) of elastic fabric, at least one layer (6) of artificial hair.

7. Covering (1) according to any of the preceding claims, **characterised in that** the elastic fabric of at least one of the layers (41, 42, 43, 44, 45, 46) of elastic fabric is a mesh fabric comprising at least a polyamide.

8. Covering (1) according to any of the preceding claims, **characterised in that** at least one or preferably each layer (41 to 46) of elastic fabric has a thread count in the range of from 1×10⁻³ to 3.33×10⁻³ g/m, preferably 1.33×10⁻³ to 2.77×10⁻³ g/m.

9. Covering (1) according to any of the preceding claims, **characterised in that** since said covering (1) is a covering for the body of a femoral prosthesis, said covering (1) comprises, in the area situated above the knee, a fold (7) for reserve stretching.

10. Limb prosthesis comprising at least one prosthesis body (10) covered by a covering (1) which can be attached to said body (10), **characterised in that** said covering (1) is according to any of claims 1 to 9.

11. Method for manufacturing a covering (1) for a limb prosthesis body (10) according to any of claims 1 to 9, which is intended to dress said body (10), **characterised in that** said method comprises at least:
a) a step during which at least one layer (45, 46) of elastic fabric in the form of a sleeve is attached to a positive mould of the limb to be reconstructed,
b) a step during which the layer (45) of elastic fabric last attached is covered with a layer (3) of silicone,
and **in that** step a) is repeated at least twice and step b) at least once in order to obtain a superposition of layers comprising, from the outside towards the inside of the covering (1), at least one external layer (2) of transparent or translucent silicone, at least two layers (41, 42, 43, 44) of elastic fabric, a layer (3) of silicone and at least one layer (45, 46) of elastic fabric.
